# FASCICULE DE BREVET EUROPEEN

(11) **EP 3 923 836 B1**
(45) Date de publication et mention de la délivrance du brevet: **25.09.2024**
(21) Numéro de dépôt: 20712633.5
(22) Date de dépôt: 10.02.2020
(51) Int. Cl.: A61B 17/44

(54) **SPATULE OBSTETRICALE ARTICULABLE**
GELENKIGE GEBURTSSPATEL
ARTICULATABLE OBSTETRIC SPATULA

(30) Priorité: 13.02.2019 FR 1901416
(43) Date de publication de la demande: 22.12.2021
(73) Titulaire: Ami, Olivier, 75001 Paris (FR)
(72) Inventeur: Ami, Olivier, 75001 Paris (FR)
(74) Mandataire: Demulsant, Xavier
(86) Numéro de dépôt international: PCT/FR2020/050241
(87) Numéro de publication internationale: WO 2020/165535

(56) Documents cités:
- CN-U- 205 163 213
- FR-A1- 2 761 875
- US-A- 1 072 038
- US-A- 5 849 017

## Description

La présente invention se rapporte aux instruments d'extraction pour accouchement par voie basse ou césarienne.

La présente invention concerne une spatule obstétricale articulable, qui fournit un moyen confortable, rapide et sûr pour effectuer une extraction instrumentale foetale pendant la deuxième phase du travail par voie vaginale et / ou une césarienne.

En 2010, en France, 12% des naissances impliquaient le recours à une extraction instrumentale : 5.3% par ventouse, 3.9% par forceps, et 2.9% par spatule (Blondel et al, Les naissances en 2010 et leur évolution depuis 2003, Enquête nationale périnatale, mai 2011*).*

Cette situation restait la même en 2016, la ventouse représentant comme en 2010 le principal instrument utilisé par les obstétriciens en cas de réalisation d'une extraction instrumentale, devant le forceps et les spatules (respectivement 27.6 % et 22.6% des naissances par voie instrumentale, Blondel et al, Les naissances et les établissements situation et évolution depuis 2010, Enquête nationale périnatale, octobre 2017)*.*

Parmi les complications néonatales des forceps, les plus fréquentes sont les ecchymoses et les abrasions cutanées. Toutefois, plus rarement, on observe des céphalhématomes, des hématomes sous-galéaux, des fractures du crâne, des hémorragies intracrâniennes, des lésions oculaires, des paralysies faciales et du plexus brachial, des traumatismes médullaires cervicaux.

Quant aux spatules, les lésions foetales dues à leur utilisation sont généralement rares et bénignes.

En ce qui concerne les complications maternelles immédiates, certaines sont communes aux deux instruments (déchirures vulvo-périnéales et cervico-vaginales). D'autres sont plus liées à l'utilisation du forceps (rupture utérine, thrombus des espaces péri-génitaux, plaie vésicale ou de l'urètre, plaie sphinctérienne, anale ou rectale basse). Certains auteurs indiquent des risques de traumatisme du vagin lors de l'emploi de spatules, en particulier atteinte du plexus brachial par pression du bec des spatules sur les régions sus-claviculaires et cervicales (Merger et al, Précis d'obstétrique, 6ème édition, ISBN 978-2-294-00897-9)*.*

### L'état de la technique concernant les forceps peut être résumé de la manière suivante.

Le forceps est un instrument, en général métallique, en forme de pinces, qui permet de saisir la tête d'un foetus pour faciliter son expulsion, le forceps permettant une traction.

Les indications d'extraction instrumentale par forceps peuvent être posées soit dans l'intérêt foetal (anomalie du rythme cardiaque foetal, survenue rapide d'une acidose foetale, anomalies de variétés, défaut de progression du mobile foetal), soit dans l'intérêt maternel (limitation des efforts expulsifs, notamment pour les femmes porteuses d'une cicatrice utérine, ou souffrant d'une maladie telle qu'éclampsie, cardiopathie, insuffisance respiratoire), ces deux types d'indications pouvant être associées.

Le forceps aurait été inventé au XVlème siècle en Angleterre par Peter Chamberlain. Il ne comporte alors qu'une seule courbure, dite courbure céphalique. André Levret, accoucheur de la mère de Louis XVI, en introduit l'usage en France et le modifie en introduisant une seconde courbure aux cuillères, dite courbure pelvienne, une illustration de son forceps étant donnée dans ses *Observations sur les causes et les accidents de plusieurs accouchements laborieux, seconde édition, 427 pages, Paris 1750.*

Les principales évolutions des forceps datent de la fin du XIXème siècle au début du XXème siècle. En 1861, Pajot propose un forceps à branches croisées. En 1877, Stéphane Etienne Tarnier améliore le forceps en lui ajoutant un tracteur, permettant d'exercer une traction sur la tête de l'enfant suivant l'axe de l'excavation pelvienne maternelle. En 1899, Lucien Alfred Démelin propose un forceps à branches parallèles, pour réduire la compression sur la tête foetale, une variante étant proposée étant le forceps de Suzor. En 1916, le norvégien Christian Caspar Gabriel Kielland propose un forceps à articulation mobile (voir document DE349104).

Selon Lansac et al (Extractions instrumentales : forceps, spatules, ventouses, Pratique de l'accouchement, ISBN 978-2-194-74776-2, pages 347- 375), sept cent types de forceps sont utilisés dans le monde, sans qu'aucune étude n'ait montré la supériorité de l'un ou de l'autre, deux grands types de forceps étant proposés : les forceps à branches croisées (Tarnier, Pajot), et les forceps à branches convergentes (Demelin-Suzor).

Une présentation des évolutions des forceps depuis leur création est donnée par Bryan Hibbard The obstretrician armamentarium, Historical obstetric instruments and their inventors, 2000, ISBN10:0930405803*).*

Une description des principaux forceps est présentée par Féraud (Forceps : description, mécanique, indications et contre-indications, Journal de Gynécologie Obstétrique et Biologie de la Reproduction 2008, 37, S202-209*).*

Une présentation des différents types de forceps et une proposition de classification est donnée en 2005 par Dupuis et al, Les forceps hier, aujourd'hui et demain, une nouvelle classification des forceps, Gynécologie Obstétrique & Fertilité 33, pp. 980-985*.* Selon ces auteurs, plus de quatre cents types de forceps aurait été décrits dans la littérature. La classification proposée distingue, pour les forceps opératoires : les forceps croisés sans tracteur, avec articulation fixe (*Levret, DeeLee, Simpson, Pajot*), ou avec articulation mobile (*Kielland*) ; les forceps croisés avec tracteur (*Tarnier*) ; les forceps à branches parallèles (*Démelin-Suzor, Gilles, Shute*)); les spatules inventées par Thierry en 1950, considérées comme un instrument de propulsion et non de traction.

Cette classification comprend par ailleurs des forceps expérimentaux, utilisés in vitro ou lors d'études de recherche cliniques, ces forceps étant instrumentés. Une grande variété de capteurs a été proposée : dynamomètres permettant de mesurer la force de traction maximale utilisée, jauges de contraintes, capteurs hydrogonflables, capteur de pression (voir par exemple les documents US3785381, US5649934, US6425899, WO2004108010, WO2010043860, WO2011131988, WO2019086904, CN204931814).

Dans la plupart des forceps proposés dans l'art antérieur, les cuillères comportent un évidement dénommé fenêtre, constituée d'une branche antérieure, dite jumelle antérieure, et d'une jumelle postérieure, réunies en avant au niveau du bec.

L'on connait également des forceps à cuillères pleines, dépourvues de fenêtres (forceps de Luikart, de Tucker-Mc Lane). Le document US3088465 (Smith) décrit un forceps croisé à branches séparables et cuillères pleines, dépourvues de fenêtres.

Différents moyens ont été présentés pour tenter de limiter les complications lors de l'emploi de forceps.

Pour tenter de limiter l'apparition de complications lors de l'emploi de forceps, l'enseignement du forceps opératoire avec des simulateurs a été tenté par le passé, par exemple BirthSIM (Moreau et al, The automated BirthSIM simulator for the obstetrician training, APII-JESA Journal Européen des Systèmes Automatisés, 2011, vol.45, no.4-6, pp. 415-35 *;* Dupuis et al, Assesment of forceps blade orientations during their placement using an instrumented childbirth simulator, BJOG An International Journal of Obstetrics & Gynaecology. Jan2009, Vol. 116 Issue 2, p327-333)*.*

Pour tenter de réduire les efforts exercés sur la tête du foetus, il a été proposé de réaliser les branches de forceps dans d'autres matériaux que l'acier, ou de placer des inserts de protection en face interne des cuillères.

Le document US5849017 (Reynolds) décrit un forceps croisé à branches séparables, les branches étant en polycarbonate renforcé de fibres de verre, les cuillères étant pleines, dépourvues de fenêtre, la surface interne des cuillères étant pourvue d'une texture et n'étant pas lisse.

Le document US5674243 (Hale) décrit un forceps croisé à branches séparables, les cuillères étant pleines et dépourvues de fenêtre, les branches étant en polyuréthane, par exemple fabriquées par injection, et comprenant de préférence un insert en acier. Le document Hale et al, A new obstetrical polyurethane versus stainless steel forceps : a comparison of forces generated to the base of the fetal skull during simulated deliveries J Perinat Med 37, pp.669-671 présente les résultats d'une simulation des performances de ce type de forceps.

Le document DE4235442 (Werner) décrit un forceps croisé dont les cuillères portent, en face interne, des inserts gonflables. Un autre exemple d'une telle disposition est présenté dans le document US2637320 (Greenberg).

Le document DE824670 (Hinselmann) décrit un forceps croisé dont les cuillères portent, en face interne, un revêtement en caoutchouc.

Le document FR 3025087 décrit un extracteur foetal comprenant deux instruments distincts terminés respectivement par deux parois creuses de forme adaptée pour être appliquée sur le crâne du foetus, chaque paroi comprenant une paroi externe convexe et une paroi interne concave, entre lesquelles est délimité un interstice raccordé à un orifice de raccordement, permettant le branchement vers un moyen d'aspiration tel qu'une seringue, la paroi interne concave étant traversée d'au moins un orifice communiquant avec l'interstice. Les orifices forment ainsi des ventouses, conduisant la peau crânienne du foetus à adhérer sur les parois internes des deux parois creuses des instruments, procurant la force de préhension nécessaire à l'extraction du foetus.

### L'état de la technique concernant les spatules peut être présenté comme suit.

Les spatules sont parfois présentées comme des forceps non articulés (voir par exemple Boucoiran et al, Spatula-assisted deliveries : a large cohort of 1065 cases, European Journal of Obstetrics & Gynecology and Reproductive Biology, 151, 2010, 46-51*).* La classification des forceps proposée par *Dupuis et al* place les spatules de Thierry parmi les forceps opératoires.

Toutefois, contrairement au forceps, les leviers des anciens accoucheurs, remplacés actuellement par les spatules, sont des instruments d'orientation de la tête foetale et de propulsion, et non de préhension et de traction.

La force de traction appliquée sur le manche des spatules est transformée, par l'intermédiaire d'un appui sur les parties molles maternelles et sur l'os malaire foetal, en une force de propulsion de la tête foetale. Chacun des deux leviers prend appui par l'extrémité de sa face interne sur la région malaire foetale, et prend appui par sa face externe sur le périnée maternel.

Les spatules agissent sur la tête foetale en permettant d'écarter les tissus maternels. Les spatules sont souvent présentées comme les prolongations des mains de l'accoucheur. Le point d'application du levier se fait spontanément au tiers moyen de la face convexe des spatules. Dès que la traction commence en écartant légèrement les manches, l'extrémité des spatules propulse la présentation en même temps que la résistance des tissus maternels est diminuée. Est ainsi associée à la force de traction, qui reste réduite, une force de propulsion due à un effet de levier.

Deux types de spatules ont été commercialisés en France (Simon-Toulza, Spatules : description, mécanique, indications et contre-indications, Journal de Gynécologie Obstétrique et Biologie de la Reproduction, 2008, 37, S222-S230).

En 1950 apparaissent les spatules proposées par le docteur Emile Jean-François Thierry (Nouvel instrument destiné à remplacer le forceps, La Presse Médicale 1950, 81, pages 1423-1427*,* voir également les brevets FR993416 et FR1228043). Elles furent créées afin d'éviter les lésions traumatiques foetales provoquées par les forces, dues au couple mécanique céphalo-instrumental, lors de la solidarisation des deux branches des forceps autour de la tête.

En 1971 apparaissent les spatules proposées par Georges Teissier (G. Teissier, P. Miliani, C. Guidicelli, and J. Jubelin, Présentation d'un nouveau type de spatules. Bulletin de la Fédération des Sociétés de Gynécologie et d'Obstétrique de Langue Française, pp.343-344, 1971)*.*

Les spatules de Thierry sont composées de deux leviers indépendants, symétriques, sans perforation, sans articulation, sans moyen de serrage telle que des vis, chaque levier comprenant un manche et une cuillère. Les spatules de Thierry ne comportent ni jumelles ni cavités pour les lacs. Les spatules de Thierry ne comportent pas de courbure céphalique, et leur courbure faciale est à plus grand rayon que la courbure céphalique des forceps. Le plan des poignées est parallèle à l'axe des spatules, contrairement aux forceps. La cuillère est d'une largeur de 5 cm environ et une longueur de 16 cm environ, et est légèrement concave, pour s'adapter à la courbure céphalique du foetus. Des crans sur le manche permettent de positionner les doigts. Les spatules Thierry les plus courantes ont une longueur d'environ 36 cm et pèsent chacune environ 365 grammes.

Les spatules de Teissier sont plus courtes. Leur maniement et propriétés sont proches de celles des spatules Thierry et elles sont présentées comme une variante.

La diffusion des spatules de Thierry semble limitée en raison notamment d'une faible diffusion de l'apprentissage en dehors de sa zone de création (El Haloui, Evaluation de la courbe d'apprentissage des extractions par spatules de Thierry, thèse faculté de médecine de Nice, 2013)*.*

Au début des années 2000, les spatules de Thierry n'étaient utilisées qu'en France et étaient quasiment inconnues dans le reste du monde, à l'exception de l'Espagne et de quelques pays d'Amérique du Sud (De Troyer et al, Extraction instrumentale par spatules de Thierry, J Gynecol Obstet Biol Reprod 2005, 34, 795-801).

La diffusion des spatules de Teissier est encore plus confidentielle.

Les spatules de Thierry sont parfois utilisées en France en cas de variété postérieure persistante, pour une rotation instrumentale, avec possibilité de retirer les cuillères après rotation pour permettre un accouchement spontané.

Un exemple ancien de spatule obstétricale peut être trouvé dans le document US1072038 (Plummer), publié en 1913. Dans une conception encore plus ancienne, Jean Palfyn propose en 1721 un instrument dénommé « mains de fer », comprenant deux spatules à branches parallèles et manche en bois, chaque spatule comprenant une cuillère non fenêtrée. L'on peut se référer également aux documents FR993416 (Thierry) et FR128043 (Thierry).

Le document US3776240 (Woodson) décrit une spatule obstétricale en acier inoxydable, comprenant une tête concave, formée d'une bande pleine, et un manche cylindrique lisse. Un autre exemple de spatule obstétricale est présenté dans le document US4136679 (Martinez).

Le document ES1042774 (Verges) décrit des spatules obstétricales réalisées en matériau polymère renforcé de fibres de carbone.

Les forceps et spatules sont ainsi utilisés de manière égale, en France, alors même que les complications liées à leurs emplois ne sont pas égales.

Dans une étude datant de 2012, à propos de 77 cas, il était conclu que les spatules de Thierry, comme le forceps, constituent un moyen sûr et efficace d'extraction foetale, les spatules assurant une protection foetale relative, les ecchymoses étant plus fréquentes lors de l'emploi de forceps, les lésions périnéales sévères n'étant pas plus souvent observées avec les spatules qu'avec les forceps (Tsoyem Mouafou et al, Gynécologie Obstétrique & Fertilité March 2014 42(3):144-148).

Dans une étude datant de 2016, à propos de 142 cas, il était conclu que l'utilisation des spatules de Thierry est moins traumatique pour l'enfant que l'utilisation de forceps. D'un point de vue maternel, l'utilisation de spatules donne lieu à un taux de périnée intact plus élevé et à moins de lésions périnéales sévères que l'utilisation de forceps (Durand-Maison et al, Spatules de Thierry ou forceps : comparaison des morbidités materno-fœtales, Gynécologie Obstétrique & Fertilité January 2016 44(1):17-22)*.*

Dans une étude publiée en 2018, concernant 3710 naissances par voie basse, il est conclu que les risques de dommage du sphincter anal après utilisation de spatules de Thierry ou de forceps sont semblables (Veira et al, Thierry's spatula : a greater risk factor for obstetric anal sphincter injuries, European Journal of Obstetrics & Gynecology & Reproductive Biology. Mar2019, Vol. 234, pe89-e90).

Au total, l'extraction instrumentale foetale est traditionnellement effectuée à l'aide de forceps (par exemple forceps de Kjelland), perforés, articulés et utilisés pour la traction et, de spatules de Thierry qui ne sont pas perforées, ne sont généralement pas articulées et servent à faire glisser le foetus comme un chausse-pied.

Dans certains cas, ces dispositifs de traction du foetus sont très agressifs et dans d'autres cas ces instruments coulissants sont très peu pratiques pour l'utilisateur.

La spatule obstétricale articulable selon l'invention permet de remédier à ces inconvénients.

Bien que les spatules proposées depuis seulement quelques décennies présentent des inconvénients moindres, pour la mère comme pour le foetus, que les forceps conçus il y a plus d'un siècle, les spatules restent peu utilisées, voire moins utilisées que les forceps.

Bien que plusieurs centaines de forceps soient utilisés, les lésions foetales restent fréquentes, en particulier les blessures cutanées (marques de pression), les lésions de la face, pouvant atteindre le globe oculaire. L'on observe plus rarement des lésions nerveuses périphériques, notamment celles du nerf facial, des lésions du périoste. Sont observés également des céphalhématomes, des lésions de la boîte crânienne. Ces complications sont de plus en plus objet d'actions en justice.

Il existe un besoin pour des outils mécaniques d'aide à l'accouchement, ne présentant pas les inconvénients des outils d'extraction instrumentale de l'art antérieur et permettant de raccourcir la période d'expulsion, d'aider à la flexion et/ou compléter la rotation de la tête foetale, de réduire les risques d'incidents et de morbidité pour la mère et le foetus.

Il existe un besoin pour de tels outils mécaniques d'assistance à l'accouchement dont l'emploi doit permettre de réduire les risques de contentieux, d'améliorer le bien-être foetal, de réduire le taux de césarienne pendant le travail, d'améliorer l'image des instruments obstétricaux et de réduire les frais de prise en charge de l'accouchement.

Il existe un besoin pour de tels outils mécaniques d'assistance à l'accouchement dont l'emploi ne doit pas entraîner un apprentissage long et difficile.

A ces fins, il est proposé une spatule obstétricale comprenant un manche s'étendant suivant une direction sensiblement longitudinale et une cuillère délimitée par un bord libre et comprenant une face interne concave et une face externe, le bord libre de la cuillère comprenant une partie extrême centrale et deux parties latérales courbées, la partie extrême centrale s'étendant suivant une direction sensiblement transversale, la face interne de la cuillère étant pourvue d'au moins un relief s'étendant entre deux gorges, le relief comprenant une face proximale et une face distale, la face distale étant orientée vers le bord libre de la cuillère, la face proximale étant orientée vers le manche.

Contrairement aux spatules de *Thierry* ou de *Teissier* connues dans l'art antérieur, et contrairement aux branches de forceps connus dans l'art antérieur qui sont soit pourvus d'une fenêtre bordée de jumelles (forceps de *Tarnier, Pajot, Démelin-Suzor, Naegele, Kielland, DeLee, Simpson, Elliot, Wrigley, Boerma, Piper, Bamberg, Dewey, Barton*) ou pourvus d'une cuillère lisse (forceps de *Luikart, Tucker-Mc Lane*), la présente spatule obstétricale comprend une cuillère sans fenêtre et n'est pas pourvue d'une cuillère lisse.

Tout au contraire, la cuillère comporte un relief s'étendant entre deux gorges, le relief comprenant une face proximale et une face distale, la face distale étant orientée vers le bord libre de la cuillère, la face proximale étant orientée vers le manche.

L'orientation de cette face distale et de cette face proximale permettent d'introduire de manière atraumatique la spatule dans le site opératoire. L'orientation de la face distale assure que la spatule n'est pas blessante lorsqu'elle sert de guide pour le passage du foetus.

Avantageusement, au moins une des deux gorges est pourvue d'au moins un trou traversant débouchant en face externe et en face interne de la cuillère.

Cette disposition facilite le drainage des fluides qui peuvent se trouver autour du bébé. Dans certaines mises en oeuvre, chaque gorge est pourvue de plusieurs trous traversant, les trous étant par exemple disposés à égale distance les uns des autres, sur toute l'étendue de chaque gorge.

Avantageusement, les trous ont un diamètre d'ouverture compris entre 0.7mm et 1.9 mm. Le drainage des fluides pouvant se trouver autour du bébé s'effectue notamment par capillarité.

Avantageusement, la face proximale et la face distale du relief sont reliées par un bord comprenant une partie centrale, s'étendant suivant une direction sensiblement transversale, et deux parties latérales courbées. Le bord est avantageusement arrondi, le relief étant dépourvu d'arête vive.

Avantageusement, la face proximale et la face distale du relief sont reliées par un bord s'étendant à une distance sensiblement constante du bord libre de la cuillère.

Avantageusement, la cuillère comprend une portion centrale, de profondeur maximale, et une portion périphérique s'étendant autour de la portion centrale, les deux gorges et le relief s'étendant dans la portion périphérique.

Avantageusement, la cuillère comprend au moins deux reliefs s'étendant à égale distance l'un de l'autre.

Avantageusement, la cuillère comprend deux reliefs, la face distale du premier relief et la face proximale du deuxième relief débouchant sur la même gorge.

Avantageusement, la cuillère est pourvue d'un plan de symétrie axiale.

Il est également proposé un ensemble comprenant deux spatules telles que présentées ci-dessus, chaque spatule comprenant une section intermédiaire s'étendant entre le manche et la cuillère, chaque spatule étant pourvue, dans sa section intermédiaire, d'un dispositif permettant la solidarisation en liaison articulée des deux spatules.

Contrairement aux conceptions de l'art antérieur, dans lesquelles les spatules sont des outils distincts et concurrents des forceps, il est proposé une spatule pouvant être utilisée en aide à l'accouchement, comme moyen de propulsion, ou pouvant être utilisé dans un ensemble de deux spatules formant forceps obstétrical.

La spatule est articulable et l'assemblage de deux spatules permet d'obtenir un forceps obstétrical. La disposition des parois proximales, dans chacune des deux cuillères, assure une prise ferme lorsqu'un effort de traction est effectué sur le forceps.

L'ensemble comprenant les deux spatules, articulées entre elles, fournit un moyen confortable, rapide et sûr, pour effectuer une extraction instrumentale foetale pendant la deuxième phase du travail, par voie vaginale et/ou en césarienne.

Avantageusement, chaque spatule comprend une structure de renfort, s'étendant dans la section intermédiaire, entre la cuillère et le dispositif permettant la solidarisation en liaison articulée des deux spatules.

Avantageusement, au moins une des deux spatules est réalisée en un matériau polymère ou un matériau polymère renforcé de fibres. Par exemple, les spatules sont réalisées en polyuréthane, en polymère chargé de fibre de verre, en polymère chargé de fibre de carbone.

La spatule se compose d'une cuillère et d'une poignée conçues avec une géométrie et des mesures précises pour le bon fonctionnement de l'appareil.

La cuillère de la spatule est compacte et remplie, ou non ajourée, pour éviter les blessures foetales.

Dans sa concavité et dans la partie supérieure, la cuillère contient des ondulations biseautées, qui permettent d'introduire le passage atraumatique de la spatule dans une direction dans le site opératoire et une prise ferme pour l'enlever et ainsi la déplacer dans la direction opposée.

Dans toute son épaisseur, à l'intérieur des ondulations et dans la concavité de la cuillère elle-même, il y a des trous, avantageusement de 0,7 à 1,6 mm de diamètre, qui servent de drainage par capillarité pour les fluides qui peuvent entourer le bébé.

Autour du centre de la concavité, les trous sont avantageusement disposés en géodes hexagonales, pour éviter la fragilisation de la structure.

Le manche de la spatule est composé de la partie active ou grip en bas et de la tige en haut qui relie la cuillère à la partie grip du manche.

La poignée a la particularité de contenir d'un côté une encoche où l'on peut introduire une autre spatule exactement identique, pour obtenir un forceps en superposant la tige d'une spatule dans l'encoche de la partie active d'une autre spatule symétrique.

D'un côté de l'encoche se trouve la tige qui, étant fine, laisse une marge de manoeuvre à l'utilisateur et peut donc porter des structures de renforcement pour éviter les fractures.

D'autres objets et avantages de l'invention apparaîtront à la lumière de la description de modes de réalisation, faite ci-après en référence aux dessins annexés dans lesquels :
- la figure 1 représente une vue en perspective de la spatule ;
- la figure 2 représente une vue antérieure de la spatule ;
- la figure 3 représente une vue postérieure de la spatule ;
- la figure 4 représente une vue latérale de la spatule.

En référence à ces dessins, la spatule est constituée d'une cuillère 1 et son manche 2.

Le manche 2 s'étend selon une direction L sensiblement longitudinale.

La cuillère 1 est délimitée par un bord libre 10 et comprend une face interne 11 concave et une face externe 12.

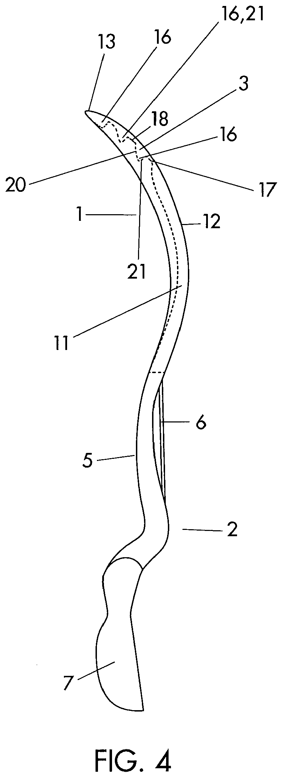

Ainsi qu'il apparait notamment en figure 4, la cuillère 1 présente une double concavité, à savoir une première concavité mesurable dans un plan longitudinal, et une deuxième concavité mesurable dans un plan transversal.

Le bord libre 10 de la cuillère 1 comprend une partie extrême 13 centrale et deux parties latérales 14, 15 courbées.

La partie extrême 13 centrale s'étend suivant une direction sensiblement transversale, perpendiculaire à la direction L longitudinale.

La face interne 11 de la cuillère 1 est pourvue d'au moins un relief 16 s'étendant entre deux gorges 17,18, le relief 16 comprenant une face proximale 19 et une face distale 20, la face distale 20 étant orientée vers le bord libre 10 de la cuillère 1, la face proximale 19 étant orientée vers le manche 2.

Dans le mode de réalisation représenté, la face interne 11 de la cuillère est pourvue de trois reliefs 16 sensiblement équidistants.

Dans d'autres modes de réalisation, non représentés, la face interne de la cuillère comprend deux reliefs, ou plus de trois reliefs, équidistants ou non.

Dans le mode de réalisation représenté, la face interne 11 de la cuillère est pourvue de trois reliefs 16 sensiblement identiques.

Dans d'autres modes de réalisation, non représentés, la face interne de la cuillère est pourvue d'au moins deux reliefs différents l'un de d'autre.

Dans le mode de réalisation représenté, la face interne 11 de la cuillère est pourvue d'un premier relief 16 disposé entre deux gorges 17, 18, un deuxième relief 21 disposé entre deux gorges 18, 22, le premier relief 16 et le deuxième relief 21 étant séparés par une même gorge 18.

Dans d'autres modes de réalisation, non représentés, la face interne de la cuillère est pourvue de reliefs ne s'étendant pas de part et d'autre d'une même gorge.

L'orientation de la face distale 20 et de la face proximale 19 de chaque relief 16 permet d'introduire de manière atraumatique la spatule dans le site opératoire.

L'orientation de la face distale 20 assure que la spatule n'est pas blessante lorsqu'elle sert de guide pour le passage du foetus.

Avantageusement, au moins une des deux gorges 17, 18 disposée de part et d'autre d'un relief 16 est pourvue d'au moins un trou traversant 30 débouchant en face externe 12 et en face interne 11 de la cuillère 1.

Cette disposition facilite le drainage des fluides qui peuvent se trouver autour du bébé.

Dans certaines mises en oeuvre, chaque gorge 17, 18, 22 est pourvue de plusieurs trous 30 traversant, les trous 30 étant par exemple disposés à égale distance les uns des autres, sur toute l'étendue de chaque gorge 17, 18, 22.

Avantageusement, les trous 30 ont un diamètre d'ouverture compris entre 0.7mm et 1.9 mm.

Le drainage des fluides pouvant se trouver autour du bébé s'effectue notamment par capillarité.

Avantageusement, la face proximale 19 et la face distale 20 de chaque relief 16 sont reliées par un bord 31 comprenant une partie centrale 32, s'étendant suivant une direction sensiblement transversale, et deux parties latérales 33, 34 courbées.

Le bord 31 est avantageusement arrondi, les reliefs 16 étant dépourvus d'arête vive.

Avantageusement, la face proximale 20 et la face distale 19 de chaque relief 16 sont reliées par un bord 31 s'étendant à une distance sensiblement constante du bord libre 10 de la cuillère 1.

Ainsi qu'il apparait dans le mode de réalisation représenté, les bords 31 des reliefs 16 s'étendent sensiblement en arc d'ellipse, à égale distance les uns des autres.

Dans le mode de réalisation représenté, les trous 30 disposés dans les gorges 17, 18, 22 apparaissent, en face externe 12 de la cuillère 1, en une disposition en trois arcs d'ellipse, les arcs étant à égale distance les uns des autres.

Avantageusement, la cuillère 1 comprend une portion centrale 40, de profondeur maximale, et une portion périphérique 41 s'étendant autour de la portion centrale 40, les gorges 17, 18, 22 et les reliefs 16 s'étendant dans la portion périphérique 41, à proximité du bord extrême 13.

Dans le mode de réalisation représenté, la portion centrale 40 de la cuillère 1 est pourvue de trous traversant 42, disposés suivant un motif régulier, par exemple à maille hexagonale.

Cette disposition permet d'assurer un passage de fluide par capillarité, sans que la résistance mécanique de la cuillère ne soit trop réduite.

Les trous 42 ont par exemple un diamètre d'ouverture compris entre 0.7mm et 1.9 mm. Les trous 42 disposés en portion centrale 40 de la cuillère 1 peuvent être de forme et ouverture identiques ou non à celles des trous 30 disposés dans les gorges 17, 18, 22.

Dans le mode de réalisation représenté, la cuillère 1 est pourvue d'un plan de symétrie axiale. Par cette disposition, une spatule peut être utilisée indistinctement comme spatule gauche pour main gauche, ou spatule droite pour main droite.

Dans la cuillère 1, les reliefs 16 et gorges 17, 18, 22 forment des ondulations biseautées 3 et les trous 42 forment trous de drainage 4 répartis au centre de la cuillère 1 en géodes hexagonales.

Au niveau de la poignée 2, la tige 5 est accompagnée d'une structure de renfort 6 et de la partie de prise 7 où se situe une encoche 8.

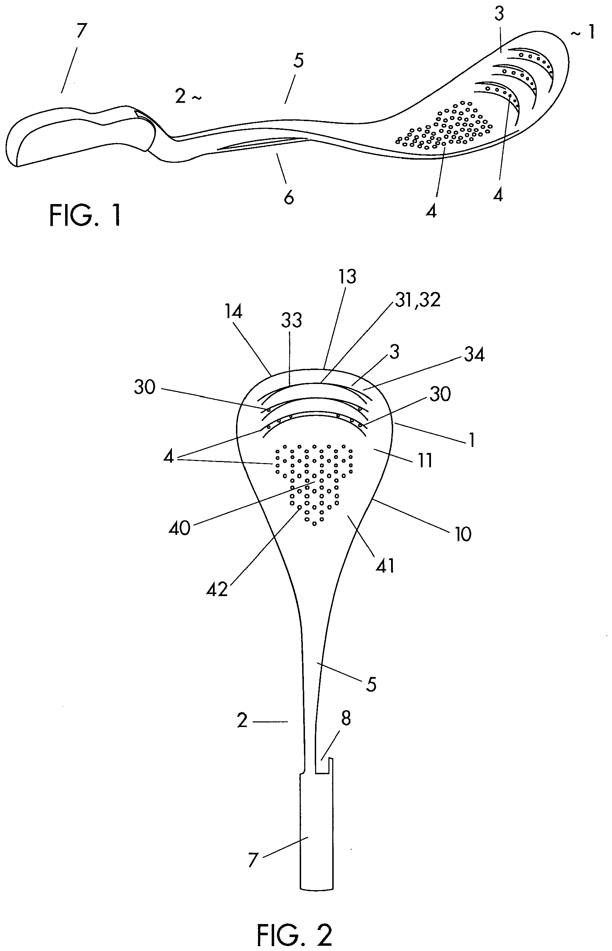

Dans la forme de réalisation, dans la figure 1, la spatule est visible en perspective avec sa cuillère 1 et son manche 2.

Dans la cuillère 1 des ondulations biseautées 3 sont présentes en périphérie, et des trous de drainage 4 sont répartis au centre de la cuillère 1 disposés en géodes hexagonales.

Au niveau du manche 2 la tige 5 s'accompagne d'une structure de renfort 6 et d'une poignée 7 permettant la prise en main.

Sur la figure 2, la face avant de la spatule est visible avec sa cuillère 1 et son manche 2.

Dans la cuillère 1 se situent les ondulations biseautées 3 en périphérie, et les trous de drainage 4 par capillarité, répartis au centre de la cuillère 1 sous forme de géode hexagonale pour ne pas fragiliser la structure.

Au niveau du manche 2 se trouve la tige 5 avec une structure de renfort 6, et la partie poignée 7 qui présente une encoche 8 permettant d'articuler avec une autre spatule identique.

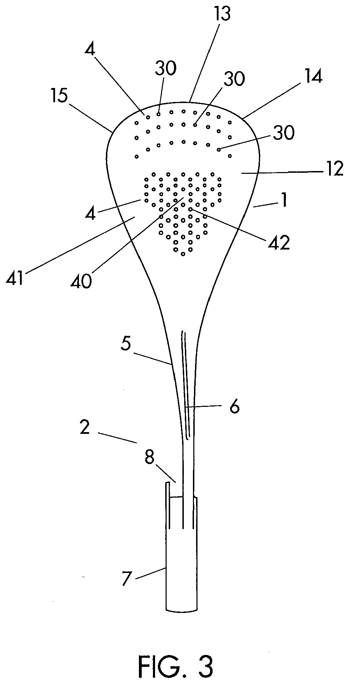

Sur la figure 3, est visible le dos de la spatule avec sa cuillère 1 et son manche 2.

Dans la cuillère 1, les ondulations biseautées 3 sont visibles avec les trous de drainage 4, répartis au centre de la cuillère 1 sous forme de géode hexagonale.

Au-dessus de la poignée 7 se situe la tige 5 avec une structure de renfort 6.

La poignée 7 est doublée d'une encoche 8.

Dans la figure 4, une vue de côté de la spatule est visible avec sa cuillère 1 et son manche 2.

Dans la cuillère 1, les ondulations biseautées 3 permettent le coulissement dans un sens et la préhension dans l'autre.

Au niveau du manche 2, la tige 5 s'accompagne d'une structure de renfort 6 et de la partie de prise 7.

À titre d'exemple non limitatif, la spatule est résistante à la stérilisation, réalisée en plastique rigide ou semi-rigide et d'aspect lisse ou granulomateux.

La spatule selon l'invention est particulièrement destinée à effectuer une extraction instrumentale foetale pendant la deuxième phase du travail par voie vaginale et / ou une césarienne.

La spatule obstétricale peut être articulée avec une spatule parfaitement identique, la tige 5 de l'une des spatules pouvant glisser dans l'encoche 8 de l'autre spatule lorsqu'elles sont placées face à face, leur permettant ainsi de s'articuler et de pivoter l'une sur l'autre.

La spatule obstétricale articulable est une spatule avec une cuillère 1 compacte et pleine pour prévenir les blessures foetales.

Dans sa concavité, elle contient des ondulations biseautées 3 qui permettent d'introduire de manière atraumatique la spatule dans une direction dans le site opératoire et une prise ferme lorsqu'on la retire, et, dans toute son épaisseur, il y a quelques petits trous 30, 42 qui servent de dispositifs de drainage par capillarité.

Le manche de la spatule 2, composé de la partie active ou grip 7 en bas et de la tige 5 en haut.

La poignée contient d'un côté une encoche 8 où l'on peut insérer une autre spatule exactement identique, transformant ainsi l'ensemble de deux spatules en un forceps en faisant chevaucher la tige 5 d'une spatule dans la tranchée 8 de la partie active d'une autre spatule symétrique lorsque celles-ci sont situées face à face.

D'un côté de l'encoche vient la tige 5 qui peut porter des structures de renfort 6 pour éviter les fractures.

La spatule selon l'invention est particulièrement destinée à effectuer une extraction instrumentale foetale pendant la deuxième phase du travail par voie vaginale et / ou une césarienne.

Avantageusement, au moins une des deux spatules est réalisée en un matériau polymère ou un matériau polymère renforcé de fibres.

Par exemple, les spatules sont réalisées en polyuréthane, en polymère chargé de fibre de verre, en polymère chargé de fibre de carbone.

La spatule obstétricale selon l'invention présente de nombreux avantages.

Elle fournit un moyen confortable, rapide et sûr pour effectuer une extraction instrumentale foetale pendant la deuxième phase du travail par voie vaginale et / ou une césarienne.

Lorsqu'utilisées non assemblées, les spatules selon la présente invention offrent tous les avantages des spatules de l'art antérieur, notamment les spatules de Thierry, en particulier des lésions foetales généralement rares et bénignes.

Lorsqu'utilisées assemblées, les spatules selon la présente invention offrent tous les avantages des forceps connus de l'art antérieur, et permettent en outre de réduire fortement les risques de lésions sur le foetus, notamment les ecchymoses et les abrasions cutanées.

Les spatules obstétricales selon l'invention pouvant être utilisées tant comme spatules que comme forceps, leur utilisation permet d'importantes économies.

En premier lieu, il n'est plus nécessaire de disposer de jeux d'instruments distincts (spatules de Thierry d'une part, forceps d'autre part), réduisant les frais d'achat, stockage et stérilisation du matériel.

En deuxième lieu, l'apprentissage de l'utilisation des spatules et des forceps est accéléré, les personnels en formation ayant plus d'opportunité de se familiariser avec les gestes d'extraction instrumentale, à l'aide d'une seule paire d'outils. Cette familiarisation est facilitée par le fait que l'emploi des spatules est atraumatique. Le cas échéant, les spatules pourront être utilisées uniquement non assemblées, l'accouchement se poursuivant par voie basse. Dans d'autres utilisations, les spatules seront utilisées séparées, et en cas d'insuccès de la propulsion, les spatules seront ensuite assemblées pour être utilisées en forceps.

En troisième lieu, la fabrication des spatules en un matériau polymère, éventuellement renforcé de fibres, confère à l'instrument une souplesse mécanique et un contact plus acceptables pour la mère que les forceps conventionnels métalliques.

Le cas échéant, les spatules peuvent être à usage unique, facilitant leur emploi comme spatules ou assemblées n forceps dans des situations ou la stérilisation des instruments obstétricaux est difficile, couteuse, ou impossible.

L'utilisation des spatules selon la présente invention permet de raccourcir la période d'expulsion, d'aider à la flexion et/ou compléter la rotation de la tête foetale, de réduire les risques d'incidents et de morbidité pour la mère et le foetus.

L'emploi des spatules selon la présente invention permet de réduire les risques de contentieux, d'améliorer le bien-être foetal, de réduire le taux de césarienne pendant le travail, d'améliorer l'image des instruments obstétricaux et de réduire les frais de prise en charge de l'accouchement.

## Revendications

1. Spatule obstétricale comprenant un manche (2) s'étendant suivant une direction sensiblement longitudinale et une cuillère (1) délimitée par un bord libre (10) et comprenant une face interne (11) concave et une face externe (12), le bord libre (10) de la cuillère (1) comprenant une partie extrême (13) centrale et deux parties latérales (14, 15) courbées, la partie extrême (13) centrale s'étendant suivant une direction sensiblement transversale, **caractérisée en ce que** la face interne (11) de la cuillère (1) est pourvue d'au moins un relief (16) s'étendant entre deux gorges (17, 18), le relief (16) comprenant une face proximale (19) et une face distale (20), la face distale (20) étant orientée vers le bord libre (10) de la cuillère (1), la face proximale (19) étant orientée vers le manche (2).

2. Spatule obstétricale selon la revendication 1, **caractérisée en ce qu'**au moins une des deux gorges (17, 18) est pourvue d'au moins un trou (30) traversant débouchant en face externe (12) et en face interne (11) de la cuillère (1).

3. Spatule obstétricale selon la revendication 1 ou 2, **caractérisée en ce que** la face proximale (19) et la face distale (20) du relief (16) sont reliées par un bord (31) comprenant une partie centrale (32), s'étendant suivant une direction sensiblement transversale, et deux parties latérales (33, 34) courbées.

4. Spatule obstétricale selon l'une quelconque des revendications 1 à 2, **caractérisée en ce que** la face proximale (19) et la face distale (20) du relief (16) sont reliées par un bord (31) s'étendant à une distance sensiblement constante du bord libre (10) de la cuillère (1).

5. Spatule obstétricale selon l'une quelconque des revendications 1 à 4, **caractérisée en ce que** la cuillère (1) comprend une portion centrale (40), de profondeur maximale, et une portion périphérique (41) s'étendant autour de la portion centrale (40), les deux gorges (17, 18) et le relief (16) s'étendant dans la portion périphérique (41).

6. Spatule obstétricale selon l'une quelconque des revendications précédentes, **caractérisée en ce que** la cuillère (1) comprend au moins deux reliefs (16, 21) s'étendant à égale distance l'un de l'autre.

7. Spatule obstétricale selon l'une quelconque des revendications 1 à 5, **caractérisée en ce que** la cuillère (1) comprend deux reliefs (16, 21), la face distale du premier relief (16) et la face proximale du deuxième relief (21) débouchant sur la même gorge (18).

8. Spatule obstétricale selon l'une quelconque des revendications précédentes, **caractérisée en ce que** la cuillère (1) est pourvue d'un plan de symétrie axiale.

9. Ensemble comprenant deux spatules telles que présentées dans l'une quelconque des revendications 1 à 8, chaque spatule comprenant une section intermédiaire s'étendant entre le manche (2) et la cuillère (1), chaque spatule étant pourvue, dans sa section intermédiaire, d'un dispositif permettant la solidarisation en liaison articulée des deux spatules.

10. Ensemble selon la revendication 9, **caractérisé en ce que** chaque spatule comprend une structure de renfort, s'étendant dans la section intermédiaire, entre la cuillère (1) et le dispositif permettant la solidarisation en liaison articulée des deux spatules.

11. Ensemble selon la revendication 9 ou 10, **caractérisé en ce qu'**au moins une des deux spatules est réalisée en un matériau polymère ou un matériau polymère renforcé de fibres.

## Patentansprüche

1. Geburtsspatel, umfassend einen Griff (2), der sich in einer im Wesentlichen länglichen Richtung erstreckt, und einen Löffel (1), der durch einen freien Rand (10) begrenzt ist und eine konkave Innenseite (11) und eine Außenseite (12) umfasst, wobei der freie Rand (10) des Löffels (1) einen zentralen Endteil (13) und zwei gekrümmte Seitenteile (14, 15) umfasst, wobei der zentrale Endteil (13) sich in einer im Wesentlichen quer verlaufenden Richtung erstreckt, **dadurch gekennzeichnet, dass** die Innenseite (11) des Löffels (1) mit wenigstens einem Relief (16) versehen ist, das sich zwischen zwei Nuten (17, 18) erstreckt, wobei das Relief (16) eine proximale Seite (19) und eine distale Seite (20) umfasst, wobei die distale Seite (20) zum freien Rand (10) des Löffels (1) ausgerichtet ist, wobei die proximale Seite (19) zum Griff (2) ausgerichtet ist.

2. Geburtsspatel nach Anspruch 1, **dadurch gekennzeichnet, dass** wenigstens eine der beiden Nuten (17, 18) mit wenigstens einem durchgehenden Loch (30) versehen ist, das in die Außenseite (12) und in die Innenseite (11) des Löffels (1) mündet.

3. Geburtsspatel nach Anspruch 1 oder 2, **dadurch gekennzeichnet, dass** die proximale Seite (19) und die distale Seite (20) des Reliefs (16) durch einen Rand (31) verbunden sind, der einen zentralen Teil (32) umfasst, der sich in einer im Wesentlichen quer verlaufenden Richtung erstreckt, und zwei gekrümmte Seitenteile (33, 34).

4. Geburtsspatel nach einem der Ansprüche 1 bis 2, **dadurch gekennzeichnet, dass** die proximale Seite (19) und die distale Seite (20) des Reliefs (16) durch einen Rand (31) verbunden sind, der sich in einem im Wesentlichen konstanten Abstand vom freien Rand (10) des Löffels (1) erstreckt.

5. Geburtsspatel nach einem der Ansprüche 1 bis 4, **dadurch gekennzeichnet, dass** der Löffel (1) einen Mittelabschnitt (40) mit maximaler Tiefe und einen Umfangsabschnitt (41) umfasst, der sich um den Mittelabschnitt (40) erstreckt, wobei sich die beiden Nuten (17, 18) und das Relief (16) im Umfangsabschnitt (41) erstrecken.

6. Geburtsspatel nach einem der vorhergehenden Ansprüche, **dadurch gekennzeichnet, dass** der Löffel (1) mindestens zwei sich in gleichem Abstand zueinander erstreckende Reliefs (16, 21) umfasst.

7. Geburtsspatel nach einem der Ansprüche 1 bis 5, **dadurch gekennzeichnet, dass** der Löffel (1) zwei Reliefs (16, 21) umfasst, wobei die distale Seite des ersten Reliefs (16) und die proximale Seite des zweiten Reliefs (21) auf die gleiche Nut (18) münden.

8. Geburtsspatel nach einem der vorhergehenden Ansprüche, **dadurch gekennzeichnet, dass** der Löffel (1) mit einer axialen Symmetrieebene versehen ist.

9. Anordnung, die zwei Spatel umfasst, wie in einem der Ansprüche 1 bis 8 dargestellt, wobei jeder Spatel einen Zwischenabschnitt umfasst, der sich zwischen dem Griff (2) und dem Löffel (1) erstreckt, wobei jeder Spatel in seinem Zwischenabschnitt mit einer Vorrichtung versehen ist, die die Verbindung der beiden Spatel in einer Gelenkverbindung gestattet.

10. Anordnung nach Anspruch 9, **dadurch gekennzeichnet, dass** jeder Spatel eine Verstärkungsstruktur umfasst, die sich im Zwischenabschnitt zwischen dem Löffel (1) und der Vorrichtung erstreckt, die die Verbindung der beiden Spatel in einer Gelenkverbindung gestattet.

11. Anordnung nach Anspruch 9 oder 10, **dadurch gekennzeichnet, dass** wenigstens einer der beiden Spatel aus einem Polymermaterial oder einem faserverstärkten Polymermaterial hergestellt ist.

## Claims

1. An obstetric spatula comprising a handle (2) extending along a substantially longitudinal direction and a spoon (1) delimited by a free edge (10) and comprising a concave internal face (11) and an external face (12), the free edge (10) of the spoon (1) comprising a central extreme part (13) and two curved lateral parts (14, 15), the central extreme par (13) extending in a substantially transverse direction, **characterised in that** the internal face (11) of the spoon (1) is provided with at least one relief (16) extending between two grooves (17, 18), the relief (16) comprising a proximal face (19) and a distal face (20), the distal face (20) being oriented towards the free edge (10) of the spoon (1), the proximal face (19) being oriented towards the handle (2).

2. Obstetric spatula according to claim 1, **characterised in that** at least one of the two grooves (17, 18) is provided with at least one through hole (30) opening into the external face (12) and into the internal face (11) of the spoon (1).

3. Obstetric spatula according to claim 1 or 2, **characterised in that** the proximal face (19) and the distal face (20) of the relief (16) are connected by an edge (31) comprising a central part (32), extending in a substantially transverse direction, and two curved lateral parts (33, 34).

4. Obstetric spatula according to any one of claims 1 to 2, **characterised in that** the proximal face (19) and the distal face (20) of the relief (16) are connected by an edge (31) extending at a substantially constant distance from the free edge (10) of the spoon (1).

5. Obstetric spatula according to any one of claims 1 to 4, **characterised in that** the spoon (1) comprises a central part (40), of maximum depth, and a peripheral part (41) extending around the central part (40), the two grooves (17, 18) and the relief (16) extending in the peripheral part (41).

6. Obstetric spatula according to any one of the preceding claims, **characterised in that** the spoon (1) comprises at least two reliefs (16, 21) extending at equal distance from one another.

7. Obstetric spatula according to any one of claims 1 to 5, **characterised in that** the spoon (1) comprises two reliefs (16, 21), the distal face of the first relief (16) and the proximal face of the second relief (21) opening into the same groove (18).

8. Obstetric spatula according to any one of the preceding claims, **characterised in that** the spoon (1) is provided with an axial plane of symmetry.

9. Assembly comprising two spatulas such as presented in any one of claims 1 to 8, each spatula comprising an intermediate section extending between the handle (2) and the spoon (1), each spatula being provided, in its intermediate section, with a device allowing the two spatulas to be connected by an articulated link.

10. Assembly according to claim 9, **characterised in that** each spatula comprises a reinforcement structure, extending in the intermediate section, between the spoon (1) and the device allowing the two spatulas to be connected by an articulated link.

11. Assembly according to claim 9 or 10, **characterised in that** at least one of the two spatulas is made of a polymer material or a fibre-reinforced polymer material.
